# EUROPEAN PATENT SPECIFICATION

(11) **EP 0 622 051 B1**
(45) Date of publication and mention of the grant of the patent: **12.08.1998**
(21) Application number: 93118367.7
(22) Date of filing: 12.11.1993
(51) Int. Cl.: A61B 17/36

(54) **Laser light irradiation apparatus**
Laserbestrahlungsvorrichtung
Dispositif d'irradiation à laser

(30) Priority: 29.04.1993 US 53862
(43) Date of publication of application: 02.11.1994
(73) Proprietor: S.L.T. JAPAN CO, LTD., Shinjuku-ku, Tokyo 162 (JP)
(72) Inventor: Daikuzono, Norio, Ichihara-shi, Chiba-ken 290-02 (JP)
(74) Representative: Hagemann, Heinrich, Dr.rer.nat., Dipl.-Chem.

(56) References cited:
- DE-A- 3 119 322
- DE-A- 3 926 353
- US-A- 4 693 244
- US-A- 4 736 743
- US-A- 5 093 877
- US-A- 5 139 495

## Description

### Background of the Invention

### 1. field of the Invention

The present invention relates to a laser light irradiation apparatus which is used for treating the tissue of a human body or an animal body (hereinafter referred to as living tissue).

### 2. Prior Art

Irradiation of the tissue of human body with laser lights has been conducted for incision, vaporization and coagulation of thereof. Its usefullness has been recognized and widely used.

For surgical use, apparatus have heretofore been used in which the front end portion of an optical fiber is accommodated in a holder member comprising a metal sleeve and the optical fiber is held by the holder member in such a manner that the front end of the optical fiber is not projected beyond the holder member. The base end of the optical fiber is optically coupled to a laser light source so that the laser lights from the laser light source are transmitted through the optical fiber for emitting the laser light to irradiate the tissue with the laser lights.

A laser probe for irradiating tissue according to the preamble of new claim 1 is described in the DE-A-31 19 322 (**D1**). The known laser probe comprises an optical fiber whose exposed core extends into an elongated hole of a cap or a chip. In order to prevent the optical fiber from being destroyed by overheating an air gap is left between the chip and the optical fiber. A metallic ring surrounds the fiber core at a point where the clad ends. This ring is intended to improve the heat transfer from the fiber core to the air in the gap. However, no means are provided for abducting the generated heat to the outside of the chip.

The US-A-5 093 877 (**D2**) teaches an optical fiber lasing apparatus including an optical fiber which terminates with its distal end in an inner tubular channel of a lens. Preferably the distal end of the fiber is secured to the lens with epoxy adhesive. No hint is given in this reference of how to release the heat on the inner surface of the lens generated by the thermal shock of laser light to the outside of the apparatus. However, it is clear, that the heat transfer cannot take place via the epoxy adhesive which is a thermal insulator.

The US-A-5 139 495 (**D3**) and the US-A-4 736 743 (**D4**) both describe a laser probe which is mounted at the output end of an optical fiber. The laser lights being emitted from the front end of the optical fiber are incident upon the rear end of the laser probe. The fiber is optically coupled with the laser probe by way of a linking means, consisting of a male and a female connector. A passageway for water or other cooling fluids is provided coaxially around the optical fiber for the cooling of the rear end of the laser probe. The space that is needed for this coolant passage increases the size of the entire apparatus which may result in difficulties in connection with inserting the apparatus into a guide hole of an endoscope.

Surgical operations have been conducted in a non-contact manner in which the front end of the holder, that is, the optical fiber is remote from the surface of the tissue.

Since the front end of the optical fiber is remote from the object to be irradiated in case of non-contact operation, a small change in the angle of the holder may cause a large change in irradiation position. Accurate irradiation with laser lights is difficult. The laser lights emitted from the front end of the optical fiber may be attenuated in air. In order to irradiate the tissue with laser light having a necessary power, a higher power laser light generator is required. This results in an increase in the size of the irradiation apparatus and the cost of the operation.

In order to solve these problems, present inventor has proposed that laser lights be emitted from a ceramic contact chip which is disposed at the front end of the optical fiber, the laser lights being emitted from the front end of the optical fiber and incident upon the rear end of the contact chip and that an operation be conducted in such a manner that the front end of the chip is in contact with an object tissue. Such type of laser light irradiation apparatus have been widely used since higher energy can be provided for the tissue with a lower output laser light generator and the apparatus is excellent in ease of positioning of irradiation position.

If the above mentioned contact operation method is adopted, it would be necessary to optically couple the optical fiber with the chip. A structure comprising a holding member for linking an optical fiber with a chip and a holder for supporting the holding member has been proposed as is disclosed in U.S. Patent Nos. 4,592,353 and 4,736,743 issued to the preset inventor. The position of an object to be operated is changed by moving the holder gripped by an operator and by moving the holder forward or rearward externally of the body under observation through an endoscope in surgical and internal operations, respectively.

Since the laser lights emitted from the front end of the optical fiber are incident upon only the rear end of the chip, the thermal shock at the rear end face of the chip due to incident laser lights is high, cooling of the rear end of the chip with water or cooling gas externally supplied or a passage for supplying coolant is necessary.

It is therefore necessary to provide a sleeve coaxially around the optical fiber to form a coolant passage. This is particularly necessary when the output of the laser lights is increased.

This results in an increase in the size of the entire of the apparatus. Although this may be often acceptable when the apparatus is for surgical operation, it is difficult to insert the apparatus into a guide hole of an endoscope.

Accordingly, it is preferable that the sleeve has a diameter less than that of the chip.

It is not desired to concentratedly emit laser lights from the front end of the chip, but to emit around the chip in specific operation positions. It is difficult for prior art apparatus to emit enough laser lights around the chip.

### Summary of the Invention

It is therefore a first object of the present invention to provide a structure using no coolant for a chip.

It is a second object of the present invention to provide an apparatus having a smaller diameter which is particularly preferable when it is inserted into a guide hole of a endoscope.

It is a third object of the present invention to emit laser lights radially of a chip.

It is a further object of the present invention to provide an irradiation apparatus having less parts or members, which results in a reduction in cost.

In order to accomplish the object of the present invention, there is provided a laser light irradiation apparatus for irradiating a living tissue with laser lights to treat the tissue by bringing the end face of the apparatus into contact with the tissue, comprising: an optical fiber; and a substantially elongated chip disposed in front of said optical fiber for transmitting therethrough laser lights incident thereon from the front end of said optical fiber to emit the transmitted laser lights toward said object living tissue, said chip being formed with a hole extending from the center of the rear end face of the chip to a point along the length thereof, said optical fiber having an outer diameter smaller than that of said chip, said optical fiber being covered with a clad on the outer periphery of a core thereof, said clad being protected by a plastic sheath, the core being exposed along a length of the optical fiber at the front end portion thereof, said exposed portion being inserted into said hole, the rear end portion of said chip being made integral with the sheath or clad portion of the optical fiber by linking means, the apparatus being characterized in that said linking means not being substantially projected beyond the outer periphery of the chip, said linking means being formed of a stainless steel pipe and comprising at least a portion which constitutes an inner surface of said hole of said chip and a portion which constitutes a outer surface of the apparatus.

In the apparatus of the present invention, the chip is linked with the optical fiber by linking means. The linking means is not projected beyond the outer periphery of the chip. Accordingly, the laser light irradiation apparatus is provided having an outer diameter which is smaller than that of the prior art apparatus in which the outer diameter of the linking means is larger than that of the chip. The linking means may comprise a sleeve together with a heat resistant bonding agent.

The chip is provided with a hole which extends from the center of the end face thereof to a point along the length thereof. The front end portion of the optical fiber is inserted into the hole. The sheath and the clad are removed from the hole inserted portion of the optical fiber so that the core is exposed. The laser lights transmitted through the optical fiber are emitted from the outer periphery of the core as well as the front end face. The laser lights emitted from the outer periphery of the core are emitted from the side of the chip and are incident upon the object tissue. The laser lights emitted from the front end face of the core of the optical fiber are of course emitted from the front end face of the chip toward the object tissue. Accordingly, the laser lights are emitted from the entire outer surface of the chip and are incident upon the object tissue. This enables the object tissue in a wide range of area to be heated and/or incised.

An air gap having a relatively large volume is formed between the hole and the core. When the laser lights are emitted from only the front end face of the optical fiber as mentioned above, thermal shock at the rear end face of the chip due to laser lights is high. It is thus necessary to cool the rear end face of the chip with water or cooling gas externally supplied or to provide a coolant supply passage. Accordingly, with prior art probes it is necessary to provide a sleeve coaxially around the optical fiber to form a coolant passage. In contrast to this, if laser lights are emitted from the entire of the exposed core, the thermal shock at the rear end face of the chip is mitigated. Since the air gap having a comparatively large volume is formed between the hole and the core, the heat generated on the inner surface of the hole can be absorbed by the air gap if any. When emission of the laser lights is repeated, the temperature of the air in the air gap is rapidly lowered on turning off of the laser lights. This results in lowering of the temperature on the inner surface of the hole of the chip. The air gap has a function to mitigate the thermal shock on the chip. Therefore, supply of the coolant for cooling the chip becomes unnecessary, which makes it possible to reduce the size of the apparatus more.

In accordance with the present invention, reduction in the size of the laser light irradiation apparatus makes it easier to insert the apparatus into the endoscope.

An exothermic layer may be formed on the outer surface of the chip. It is preferable to use exothermic powders which generate heat on exposure to laser lights, such as carbon, graphite, iron oxide, manganese oxide and the like in order to form the exothermic layer. Since the exothermic powers are not capable of forming a coating film, it is preferable to apply the powers which are mixed with a binder. The binder is preferably heat-resistant and may include artificial or natural sapphire, quartz, glass, heat resistant plastics and the like. The coating layer may be formed by applying on the chip surface a coating liquid including exothermic powders contained in a molten binder, or a coating liquid including a binder which is dispersed in an appropriate dispersion medium such as alcohols and exothermic powers. If the binder is not molten, the coating layer may be formed by spraying the binder over the chip surface together with the exothermic powder and then heating the chip to melt it. Light scattering powders having a higher refraction index for laser lights than that of the chip may be contained in the coating layer if necessary. The light scattering powders may include artificial or natural diamond, sapphire, quartz, monocrystal zirconium oxide, high melting point glass, heat resistant plastics or the above mentioned materials which are plated with gold or aluminum on the surface thereof.

### Brief Description of the Drawings

The drawings show an irradiation apparatus of the present invention.
Fig. 1 is a schematic longitudinal sectional view showing a first embodiment of the present invention;
Fig. 2 is an enlarged front view showing an optical fiber;
Fig. 3 is a left side elevational view showing the optical fiber;
Fig. 4 is a schematic longitudinal sectional view showing a second embodiment;
Fig. 5 an enlarged longitudinal sectional view showing main part of the second embodiment;
Fig. 6 is a schematic sectional view showing a third embodiment.

### Description of the Preferred Embodiment

The present invention will become more clear from the following description of the preferred embodiments.

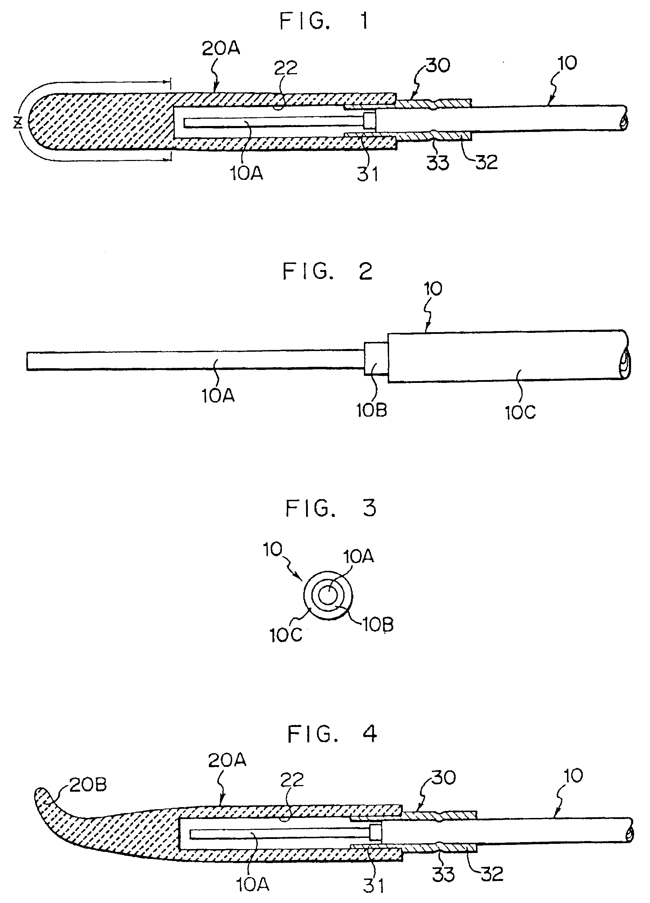

Figs. 1 to 3 show a first embodiment. A reference numeral 10 denotes an optical fiber having a core 10A and a clad 10B. The clad 10B is covered with a plastic sheath 10C on the outer surface thereof for protecting the clad. The clad 10B and the plastic sheath 10C is removed at the front end portion of the optical fiber 10 so that it is exposed. Laser lights emitted form the laser light generator (not shown) are incident upon the rear end of the optical fiber 10. The laser lights which are transmitted through the optical fiber 10 are emitted from the front end face and the outer periphery of the exposed core 10A.

A chip 20 is provided which will be in contact with an object tissue for the treatment. The chip 10 is formed of artificial or natural diamond, sapphire, quartz, monocrystal zirconium oxide, high melting point glass or heat resistant plastics and the like. The chip 20 is formed at the rear end thereof with a hole extending from the center of the rear end face 21 to a point along the length thereof toward the front end thereof. The chip 20 has an outer diameter which is larger than that of the optical fiber 10.

The core 10A portion of the optical fiber 20 is adapted into the hole 22. The chip 20 is integrally linked with the sheath 10B of the optical fiber 10 at the rear end thereof by linking means. In the first embodiment, the linking means is a heat resistant sleeve 30. The heat resistant sleeve 30 is formed of a stainless steel pipe. The sheath 10C of the optical fiber 10 is adapted into a base portion 32 of the sleeve 30 for linking the chip with the optical fiber 10. The chip 20 and the front end portion 31 of the sleeve 30 may be linked integrally with each other by pressure insertion or may be bonded with a heat resistant bonding agent such as ceramic adhesive. Linkage between the base portion 32 of the sleeve 30 and the sheath 10C of the optical fiber 10 may be achieved by caulking at 33 for bringing part of the base portion 32 into pressure contact with the sheath 10C of the optical fiber 10 as shown in Fig. 1 as well as pressure insertion. A heat generating or exothermic layer may be formed over the entire outer surface of the chip 20 or at the area Z on only the front end portion.

In the thus formed laser light irradiation apparatus, the laser lights transmitted through the optical fiber 10 are emitted from the outer periphery of the exposed core 10A portion as well as the front end face. The laser lights emitted from the outer periphery of the core 10A are emitted form the side periphery of the chip and are incident upon the object tissue to be treated. Of course, the laser lights emitted from the front end face of the core are emitted from the front end face of the chip 20 toward the object to be treated. Accordingly, the laser lights are emitted from the substantially entire outer surface of the chip 20 and are incident upon the object tissue to enable the tissue to be heated and incised over a wide range of the object tissue. If the exothermic layer is formed at the area Z as mentioned above, functions such as incision and heating of the tissue are performed at the area where the exothermic portion is formed.

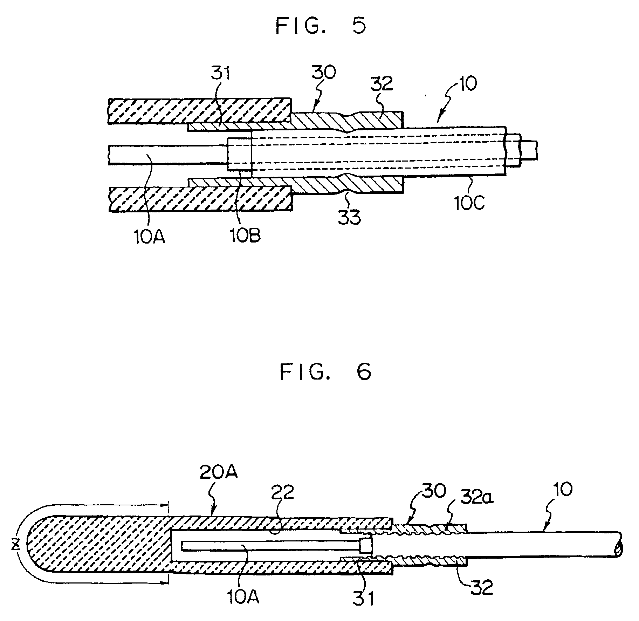

Figs. 4 and 5 show a second embodiment in which a chip 20A is formed with a bent portion 20B at the front end portion. The bent portion 20B may be formed with the above mentioned exothermic layer. The front end portion 31 of the sleeve 30 is inserted into the hole 22 for linking the same in the second embodiment. After pressure insertion of the sleeve 30, bonding may be performed with a heat resistant adhesive pressure.

Fig. 6 shows a modification of the embodiment of Fig. 1. The inner surface of the base portion 32 of the sleeve 30 is internally threaded or roughed at 32a for preventing the forcedly inserted optical fiber 10 from being removed.

In the present invention, the linking means 30 is not substantially projected beyond the outer periphery of the chip 20 or 20A.

The laser light irradiation apparatus of the present invention may be inserted into, for example, a guide hole of an endoscope and may be used for internal treatment. For surgical use, the step portion of the base portion 22 of the sleeve 30 is adapted into a front end of a sleeve-like holder (not shown) which is to be gripped by a hand.

In the present invention, the shape of the front end portion of the chip may be spherical similarly to the above mentioned embodiments, curved at the front end thereof or other appropriate shapes.

## Claims

1. A laser light irradiation apparatus for irradiating a living tissue with laser lights to treat the tissue by bringing the end face of the apparatus into contact with the tissue, comprising:
an optical fiber (10); and
a substantially elongated chip (20) disposed in front of said optical fiber for transmitting therethrough laser lights incident thereon from the front end of said optical fiber to emit the transmitted laser lights toward said object living tissue,
said chip (20) being formed with a hole (22) extending from the center of the rear end face of the chip to a point along the length thereof, said optical fiber (10) having an outer diameter smaller than that of said chip (20), said optical fiber being covered with a clad (10B) on the outer periphery of a core (10A) thereof, said clad (10B) being protected by a plastic sheath (10C), the core (10A) being exposed along a length of the optical fiber at the front end portion thereof, said exposed portion being inserted into said hole (22), the rear end portion of said chip is made integral with the sheath or clad portion of the optical fiber by linking means (30), the laser light irradiation apparatus being characterized in that said linking means not being substantially projected beyond the outer periphery of the chip (20), said linking means being formed of a stainless steel pipe and comprising at least a portion which constitutes an inner surface of said hole (22) of said chip (20) and a portion which constitutes an outer surface of the apparatus.

2. A laser light irradiation apparatus as defined in Claim 1 in which the front end portion of said chip (20) is substantially spherical in shape.

3. A laser light irradiation apparatus as defined in Claim 1 in which the front end portion of the said chip (20) is bent into the J-shape.

4. A laser light irradiation apparatus as defined in Claim 1 in which said linking means (30) comprises a thin walled heat resistant sleeve, the front end portion of said sleeve being inserted into said hole (22), said sheath of said optical fiber being adapted into the base portion of said sleeve so that said chip is linked with said optical fiber.

5. A laser light irradiation apparatus as defined in Claim 1 in which said linking means (30) comprises a heat resistant sleeve, the front end portion of said sleeve being adapted on a step portion formed on the outer periphery of the rear end of said chip, the sheath of said optical fiber (10) being adapted into base portion (32) of the sleeve so that said chip (20) is linked with said optical fiber (10).

6. A laser light irradiation apparatus as defined in Claim 4 or 5 in which the base portion (32) of the heat resistant sleeve is secured to the outer periphery of the sheath by caulking the sleeve on the sheath while the sheath of said optical fiber (10) is adapted into the base portion (32) of the heat resistant sleeve.

7. A laser light irradiation apparatus as defined in Claim 4 or 5 in which the inner surface of the base portion (32) of the heat resistant sleeve is roughed (32a) along the length thereof, said sheath of said optical fiber (10) being inserted into the roughed inner surface (32a) of the base portion (32) of said heat resistant sleeve.

8. A laser light irradiation apparatus as defined in Claim 1 or 2 in which the front end of said heat resistant sleeve is bonded to the chip (20) with a heat resistant bonding agent.

9. A laser light irradiation apparatus as defined in Claim 1 or 2 in which heat absorbing powders are applied on the outer surface of the chip (20) with a binder which transmits laser lights therethrough.

## Patentansprüche

1. Laserbestrahlungsvorrichtung zum Bestrahlen eines lebenden Gewebes mit Laserlicht, um das Gewebe durch Inkontaktbringen der Endfläche der Vorrichtung mit dem Gewebe zu behandeln, aufweisend:
eine optische Faser (10) und
einen im wesentlichen langgestreckten Chip (20), der vor der optischen Faser angeordnet ist, damit durch ihn Laserlicht, das vom vorderen Ende der optischen Faser aus auf ihn einfällt, übertragen wird, um das übertragene Laserlicht gegen das lebende Zielgewebe zu emittieren,
wobei der Chip (20) mit einem Loch (22) ausgebildet ist, das sich vom Zentrum der rückwärtigen Endfläche des Chips aus zu einem Punkt entlang der Länge desselben erstreckt, wobei die optische Faser (10) einen Außendurchmesser aufweist, der kleiner als derjenige des Chips (20) ist, wobei die optische Faser auf dem äußeren Umfang eines Kerns (10A) derselben mit einem Mantel (10B) umhüllt ist, wobei der Mantel (10B) durch eine Kunststoffhülse (10C) geschützt ist, wobei der Kern (10A) entlang einer Länge der optischen Faser am vorderen Endabschnitt derselben freigelegt ist, wobei der freigelegte Abschnitt in das Loch (22) eingefügt ist, wobei der rückwärtige Endabschnitt des Chips durch Verbindungsmittel (30) mit dem Hülsen- oder Mantelabschnitt der optischen Faser einstückig gemacht ist, wobei die Laserbestrahlungsvorrichtung dadurch gekennzeichnet ist, daß das Verbindungsmittel nicht wesentlich über den äußeren Umfang des Chips (20) hinaus vorsteht, daß das Verbindungsmittel aus einer Röhre aus rostfreiem Stahl gebildet ist und mindestens einen Abschnitt, der eine innere Oberfläche des Lochs (22) des Chips (20) bildet, und einen Abschnitt, der eine äußere Oberfläche der Vorrichtung bildet, aufweist.

2. Laserbestrahlungsvorrichtung nach Anspruch 1, bei der der vordere Endabschnitt des Chips (20) eine im wesentlichen kugelförmige Gestalt aufweist.

3. Laserbestrahlungsvorrichtung nach Anspruch 1, bei der der vordere Endabschnitt des Chips (20) J-förmig gebogen ist.

4. Laserbestrahlungsvorrichtung nach Anspruch 1, bei der das Verbindungsmittel (30) eine dünnwandige, hitzebeständige Muffe aufweist, wobei der vordere Endabschnitt der Muffe in das Loch (22) eingeführt ist, wobei die Hülse der optischen Faser in den Basisabschnitt der Muffe eingepaßt ist, so daß der Chip mit der optischen Faser verbunden ist.

5. Laserbestrahlungsvorrichtung nach Anspruch 1, bei der das Verbindungsmittel (30) eine hitzebeständige Muffe aufweist, wobei der vordere Endabschnitt der Muffe auf einen Stufenabschnitt aufgepaßt ist, der auf dem äußeren Umfang des rückwärtigen Endes des Chips ausgeformt ist, wobei die Hülse der optischen Faser (10) in den Basisabschnitt (32) der Muffe eingepaßt ist, so daß der Chip (20) mit der optischen Faser (10) verbunden ist.

6. Laserbestrahlungsvorrichtung nach Anspruch 4 oder 5, bei der der Basisabschnittt (32) der hitzebeständigen Muffe dadurch am äußeren Umfang der Hülse gesichert ist, daß die Muffe auf der Hülse dichtgestemmt wird, während die Hülse der optischen Faser (10) in den Basisabschnitt (32) der hitzebeständigen Muffe eingepaßt wird.

7. Laserbestrahlungsvorrichtung nach Anspruch 4 oder 5, bei der die innere Oberfläche des Basisabschnitts (32) der hitzebeständigen Muffe entlang der Länge derselben aufgerauht (32a) ist, wobei die Hülse der optischen Faser (10) in die aufgerauhte innere Oberfläche (32a) des Basisabschnitts (32) der hitzebeständigen Muffe eingeschoben ist.

8. Laserbestrahlungsvorrichtung nach Anspruch 1 oder 2, bei der das vordere Ende der hitzebeständigen Muffe durch ein hitzebeständiges Haftmittel mit dem Chip (20) verbunden ist.

9. Laserbestrahlungsvorrichtung nach Anspruch 1 oder 2, bei der wärmeabsorbierende Pulver mit einem Bindemittel, durch das Laserlicht übertragbar ist, auf die äußere Oberfläche des Chips (20) aufgebracht sind.

## Revendications

1. Appareil d'irradiation de lumières laser pour irradier un tissu vivant avec des lumières laser pour traiter le tissu en amenant la face d'extrémité de l'appareil en contact avec le tissu, comprenant :
- une fibre optique (10); et
- un embout (20) notablement allongé disposé en avant de ladite fibre optique pour transmettre à travers des lumières laser incidentes de l'extrémité avant de ladite fibre optique pour émettre les lumières laser transmises vers ledit tissu vivant objet,
ledit embout (20) étant formé avec un trou (22) s'étendant depuis le centre de la face d'extrémité arrière de l'embout jusqu'à un point de la longueur de celui-ci, ladite fibre optique (10) ayant un diamètre extérieur plus petit que celui dudit embout (20), ladite fibre optique étant couverte par une gaine (10B) sur la périphérie extérieure d'une âme (10A) de celle-ci, ladite gaine (10B) étant protégée par une enveloppe plastique (10C), l'âme (10A) étant nue sur une longueur de la fibre optique à la partie d'extrémité avant de celle-ci, ladite partie nue étant insérée dans ledit trou (22), la partie d'extrémité arrière dudit embout est rendue d'un seul tenant avec la partie de gaine ou d'enveloppe de la fibre optique par des moyens de liaison (30), l'appareil d'irradiation de lumières laser étant caractérisé en ce que lesdits moyens de liaison n'étant pas considérablement en saillie au-delà de la périphérie extérieure de l'embout (20), lesdits moyens de liaison sont formés d'un tube d'acier inoxydable et comprenant au moins une partie qui constitue une surface intérieure dudit trou (22) dudit embout (20) et une partie qui constitue une surface extérieure de l'appareil.

2. Appareil d'irradiation de lumières laser selon la revendication 1, dans lequel la partie d'extrémité avant dudit embout (20) est de forme approximativement sphérique.

3. Appareil d'irradiation de lumières laser selon la revendication 1, dans lequel la partie d'extrémité avant dudit embout (20) est incurvée en forme de (J).

4. Appareil d'irradiation de lumières laser selon la revendication 1, dans lequel lesdits moyens de liaison (30) comprennent un manchon résistant à la chaleur à parois minces, la partie d'extrémité avant dudit manchon étant insérée dans ledit trou (22), ladite enveloppe de ladite fibre optique étant adaptée dans la partie de base dudit manchon de sorte que ledit embout est lié à ladite fibre optique.

5. Appareil d'irradiation de lumières laser selon la revendication 1, dans lequel lesdits moyens de liaison (30) comprennent un manchon résistant à la chaleur, la partie d'extrémité avant dudit manchon étant adaptée sur une partie en échelon formée sur la périphérie extérieure de l'extrémité arrière dudit embout, l'enveloppe de ladite fibre optique (10) étant adaptée dans la partie de base (32) du manchon de sorte que ledit embout (20) est lié à ladite fibre optique (10).

6. Appareil d'irradiation de lumières laser selon la revendication 4 ou 5, dans lequel la partie de base (32) du manchon résistant à la chaleur est fixé à la périphérie extérieure de l'enveloppe par matage du manchon sur l'enveloppe tandis que l'enveloppe de ladite fibre optique (10) est adaptée dans la partie de base du manchon résistant à la chaleur.

7. Appareil d'irradiation de lumières laser selon la revendication 4 ou 5, dans lequel la surface intérieure de la partie de base (32) du manchon résistant à la chaleur est munie d'aspérités (32a) sur sa longueur, ladite enveloppe de ladite fibre optique (10) étant insérée dans la surface intérieure munie d'aspérités (32a) de la partie de base (32) dudit manchon résistant à la chaleur.

8. Appareil d'irradiation de lumières laser selon la revendication 1 ou 2, dans lequel l'extrémité avant dudit manchon résistant à la chaleur est collée à la plaquette (20) au moyen d'un agent de collage résistant à la chaleur.

9. Appareil d'irradiation de lumières laser selon la revendication 1 ou 2, dans lequel des poudres absorbant la chaleur sont appliquées sur la surface extérieure de la plaquette (20) avec un liant ou amalgame qui transmet au travers les lumières laser.
